# EUROPEAN PATENT APPLICATION

(11) **EP 2 727 997 A1**
(43) Date of publication of application: **07.05.2014**
(21) Application number: 12807142.0
(22) Date of filing: 29.06.2012
(51) Int. Cl.: C12N 5/04, A01H 4/00, A01N 37/42, A01P 21/00

(54) **PLANT CELL DIFFERENTIATION PROMOTER**

(30) Priority: 01.07.2011 JP 2011147647
(71) Applicant: Shiseido Company, Ltd., Tokyo 104-0061 (JP)
(72) Inventor: YOKOYAMA, Mineyuki, Yokohama-shi Kanagawa 236-8643 (JP); TAKAGI, Kazuteru, Yokohama-shi Kanagawa 236-8643 (JP); ONISHI, Noboru, Tokyo 164-0001 (JP); NAWATA, Yukiko, Tokyo 164-0001 (JP)
(74) Representative: Henkel, Breuer & Partner
(86) International application number: PCT/JP2012/066801
(87) International publication number: WO 2013/005695

(57) **Abstract**

The present invention addresses the problem of providing a plant cell differentiation promoter with which it is possible to promote differentiation from a callus to a normal adventitious embryo, or promote differentiation of an adventitious root or adventitious bud from a plant cutting, and as a result, obtain a regenerated plant with stability. The present invention provides a plant differentiation promoter comprising as the active ingredient a specific ketole fatty acid or derivative thereof.

## Description

### TECHNICAL FIELD

The present invention relates to a plant cell differentiation promoting agent comprising a ketol fatty acid having 4 to 24 carbon atoms in which a carbon atom that composes a carbonyl group and a carbon atom that bound to a hydroxyl group are in the α position (hereinafter referred to as a specific ketol fatty acid). The present invention further relates to a method for producing a regenerated plant body from a callus or plant section comprising the addition of a specific ketol fatty acid.

### BACKGROUND ART

Tissue culturing technologies utilizing the totipotency of plant bodies are indispensable for breeding targeted at increased production of homogenous and superior quality clones, regeneration of virus-free plants and production of new varieties. As a result of utilizing plant body tissue culturing technologies, callus can be proliferated, then the proliferated group can be subjected to organ differentiation into adventitious buds or adventitious roots, or differentiation into regenerated plant bodies through an adventitious embryo by altering the concentration ratio of auxins and cytokines in the medium. However, these tissue culturing technologies are known to be affected by factors, such as the basal medium and carbon source used, plant hormone, type of salt, concentration or culturing temperature, thus making it frequently difficult to stably induce adventitious embryos, adventitious buds and/or adventitious roots from a callus.

Transformation and cell fusion for transfecting higher plants with a foreign gene are carried out through the application of tissue culturing technology. For example, in the Agrobacterium transformation method, a process is carried out in which, after having infected a vegetative piece or callus with Agrobacterium, the tissue is transferred to differentiation medium while selecting cells transfected with a foreign gene. At this time, there are cases in which, depending on the plant species, transformants are not obtained due to the low level of differentiation ability despite plant cells having been transfected with the foreign gene. In addition, in the production of cell fusion hybrids by combining remotely related plants, even if cell fusion is obtained, one of the chromosomes may be lost at an intermediate stage due to the low level of differentiation ability, thereby resulting in the problem of preventing differentiation into a complete plant body. Thus, the efficiency of redifferentiating into a plant body from a callus is important in transformation and cell fusion technologies.

Several proposals have been made in the past for efficiently inducing differentiation in a short period of time. For example, Japanese Unexamined Patent Publication No. H05-219851 reports a method for redifferentiating plants of the Gramineae family by transplanting an adventitious embryo-like callus, obtained by culturing in callus growth medium containing potato dextrose agar, into differentiation medium having a reduced concentration of the main inorganic salt, Japanese Unexamined Patent Publication No. H06-153730 reports a callus differentiation method for Juncus effusus var. decipiens consisting of transplanting a proliferated callus to differentiation medium after drying to a certain degree followed by static culturing, Japanese Unexamined Patent Publication No. 2000-217457 reports a method for redifferentiating kenaf consisting of culturing by planting in pH-adjusted synthetic medium containing a cytokine-based plant hormone, and Japanese Unexamined Patent Publication No. 2000-270854 reports a method for producing a redifferentiated plant body consisting of culturing a portion of a plant body of Limonium sinuatum in medium containing picloram to induce callus formation, followed by allowing to proliferate during culturing and then culturing in medium containing cytokinins. Moreover, Japanese Unexamined Patent Publication Nos. H05-49470, H10-191966 and H10-229875 disclose plant callus cell differentiation agents obtained by culturing microorganisms belonging to the genus Enterobacter, Bacillus or Pseudomonas and then extracting from the culture broth.

However, in addition to these methods having limitations on the plant variety or physiological factors, their effects were not considered to be adequate.

On the other hand, the inventors of the present invention found that a ketol fatty acid having 4 to 24 carbon atoms in which a carbon atom that composes a carbonyl group and a carbon atom bound to a hydroxyl group are in the α position, and particularly 9-hydroxy-10-oxo-12(Z),15(Z)-octadecadienoic acid, has bud formation promoting action as well as plant activating action (Japanese Unexamined Patent Publication No. H11-29410 and Japanese Unexamined Patent Publication No. 2001-231506).

### DISCLOSURE OF THE INVENTION

### Problems to be Solved by the Invention

An object of the present invention is to provide a plant cell differentiation promoting agent that promotes differentiation from a callus to a normal adventitious embryo, adventitious root or adventitious bud, or promotes differentiation from a plant section to an adventitious root or adventitious bud, thereby making it possible to stably obtain a regenerated plant body as a result thereof. In addition, an object of the present invention is to provide a method for efficiently acquiring a regenerated plant body by promoting differentiation from a callus induced from a portion of a plant body to an adventitious embryo, adventitious root or adventitious bud, or by promoting differentiation from a plant section to an adventitious root or adventitious bud.

### Means for Solving the Problems

As a result of conducting extensive studies on the effects of a type of plant hormone in the form of a specific ketol fatty acid, the inventors of the present invention surprisingly found that in an induction step from a callus to an adventitious embryo, a specific ketol fatty acid promotes induction from a callus to a normal adventitious embryo, thereby leading to completion of the present invention.

The present invention also relates to a plant cell differentiation promoting agent characterized in containing the specific ketol fatty acid or a derivative thereof as an active ingredient, and a method for producing a regenerated plant body by using the specific ketol fatty acid or a derivative thereof.

More specifically, the present application includes the inventions indicated below.
[1] A plant cell differentiation promoting agent comprising a ketol fatty acid having 4 to 24 carbon atoms, wherein a carbon atom that composes a carbonyl group and a carbon atom that bound to a hydroxyl group are in the α position.
[2] The plant cell differentiation promoting agent according to [1], wherein carbon-carbon double bonds are present at 1 to 6 locations in the ketol fatty acid (provided that the number of double bonds does not exceed the number of carbon bonds of the ketol fatty acid).
[3] The plant cell differentiation promoting agent according to [1] or [2], wherein the number of carbon atoms of the ketol fatty acid is 18, and carbon-carbon double bonds are present at 2 locations.
[4] The plant cell differentiation promoting agent according to any of [1] to [3], wherein the ketol fatty acid is 9-hydroxy-10-oxo-12(Z),15(Z)-octadecadienoic acid (hereinafter referred to as KODA).
[5] The plant cell differentiation promoting agent according to any of [1] to [4], wherein the plant cell differentiation promoting agent promotes differentiation from a plant callus to an adventitious embryo, an adventitious root or an adventitious bud.
[6] The plant cell differentiation promoting agent according to any of [1] to [4], wherein the plant cell differentiation promoting agent promotes differentiation from a plant section to an adventitious bud or an adventitious root.
[7] The plant cell differentiation promoting agent according to any of [1] to [4], wherein the plant cell differentiation promoting agent promotes differentiation to a regenerated plant body by going through an adventitious embryo, an adventitious root or an adventitious bud.
[8] A method for producing a regenerated plant from a callus, comprising a step for applying the plant cell differentiation promoting agent according to any of [1] to [4] to the callus during the time period from 3 weeks before transplanting the callus maintained in a subculture medium to 2 weeks after transplanting the callus to a differentiation medium.
[9] The method for producing a regenerated plant according to [8], wherein the time period is a period from 2 weeks before transplantation to differentiation medium to 1 week after transplanting to differentiation medium.
[10] A regenerated plant body produced by using the method for producing a regenerated plant according to [9].
[11] A plant culture medium containing the plant cell differentiation promoting agent according to any of [1] to [4].

### Effects of the Invention

Induction potency to a normal adventitious embryo is demonstrated by applying (dropping) the plant cell differentiation promoting agent according to the present invention onto a callus, which is an aggregation of dedifferentiated cells. Universal conditions for inducting adventitious embryo differentiation have not yet to be established. Although an adventitious embryo is induced in many cases by culturing a callus in a medium containing auxins followed by transferring to a medium not containing auxins or only containing auxins at a low concentration, even in the case of a callus obtained from the same individual or same tissue, adventitious embryo induction potency and induction efficiency differ considerably and are unstable depending on the status of the callus. Therefore, use of the plant cell differentiation promoting agent according to the present invention makes it possible to enhance and stabilize the efficiency of adventitious embryo differentiation.

### BRIEF DESCRIPTION OF THE DRAWINGS

FIG. 1 indicates the effects of KODA during differentiation from calluses of Spathiphyllum to adventitious embryos. A large number of adventitious embryos were recovered after two weeks from a KODA treatment group in comparison with a control group.
FIG. 2 is a graph showing the effects of KODA during differentiation from calluses of Spathiphyllum to adventitious embryos. The application time of KODA is shown to have an effect on induction of adventitious embryo differentiation.
FIG. 3 indicates the effects of KODA concentration on differentiation efficiency during differentiation from calluses of Spathiphyllum to adventitious embryos.
FIG. 4 is a drawing representing the number of redifferentiated plant bodies in the results of FIG. 3 in the form of a graph. The number of redifferentiated plant bodies was shown to increase in comparison with a control group at each concentration.
FIG. 5 indicates that above ground growth and below ground growth improved in a KODA treatment group.
FIG. 6 consists of drawings representing a comparison of weights measured after drying by dividing the redifferentiated plant bodies described in the results of FIG. 5 into an above ground portion and below ground portion. Weights were demonstrated to increase as a result of KODA treatment in comparison with a control.

### BEST MODE FOR CARRYING OUT THE INVENTION

The present invention relates to a plant cell differentiation promoting agent characterized in containing a specific ketol fatty acid or derivative thereof as an active ingredient. Preferably, the plant cell differentiation promoting agent according to the present invention promotes normal differentiation from a callus to an adventitious embryo. The callus further differentiates into a regenerated plant body after differentiating to an adventitious embryo. Thus, by promoting differentiation from a callus to a normal adventitious embryo, a regenerated plant body can ultimately be efficiently obtained.

In another aspect thereof, the plant cell differentiation promoting agent according to the present invention promotes organ differentiation. An example of organ differentiation is differentiation to an adventitious organ, such as an adventitious bud or adventitious root. The plant cell differentiation promoting agent according to the present invention promotes normal differentiation from a callus to an adventitious organ or normal differentiation from a portion of a plant body to an adventitious organ such as an adventitious root or an adventitious bud. In the case of having differentiated from a callus to an adventitious organ such as an adventitious bud or adventitious root, the callus further differentiates to a regenerated plant by going through the adventitious bud or the adventitious root. In the case of differentiating from a portion of a plant body to an adventitious bud or adventitious root, a regenerated plant body is obtained as a result of the adventitious bud or adventitious root appearing from a portion of the plant body.

In still another aspect, the present invention relates to a method for producing a regenerated plant from a callus or a portion of a plant body that comprises applying a solution containing the aforementioned cell differentiation promoting agent to a callus or a portion of a plant body and culturing. Preferably, in the method for producing a regenerated plant body from a callus, the aforementioned cell differentiation promoting agent is applied by dropping once or a plurality of times onto the callus during the time period from 3 weeks before transferring to differentiation medium to 2 weeks after transplant. More preferably, the aforementioned cell differentiation promoting agent is applied once or a plurality of times to the callus during the time period from 2 weeks before transplanting to differentiation medium to 1 week after transplant. In the method for producing a regenerated plant body from a portion of a plant body, a solution containing the aforementioned cell differentiation promoting agent is applied by immersing a cut end of a plant body in the solution immediately after having cut out a portion of a plant body. Instead of applying a solution containing the cell differentiation promoting agent directly to a callus or plant body, the callus or portion of the plant body can also be cultured in a medium to which the cell differentiation promoting agent has been added.

The specific ketol fatty acid contained in the plant cell differentiation promoting agent of the present invention is a ketol fatty acid having 4 to 24 carbon atoms in which an alcohol hydroxyl group and ketone carbonyl group are present in the same molecule.

In the specific ketol fatty acid, a carbon atom that composes the carbonyl group and a carbon atom bound to the hydroxyl group are preferably in the γ position and particularly preferably in the α position. In addition, in the specific ketol fatty acid, carbon-carbon double bonds are preferably present at 0 to 6 locations (provided that the number of double bonds does not exceed the number of carbon bonds of the ketol fatty acid).

In addition, the number of carbon atoms of the specific ketol fatty acid is preferably 18, and the number of carbon-carbon double bonds present therein is preferably 1 to 2.

Specific examples of the specific ketol fatty acid include 9-hydroxy-10-oxo-12(Z),15(Z)-octadecadienoic acid (to also be referred to as specific ketol fatty acid (I) or KODA), 13-hydroxy-12-oxo-9(Z),15(Z)-octadecadienoic acid (to also be referred to as specific ketol fatty acid (II)), 13-hydroxy-10-oxo-11(E),15(Z)-octadecadienoic acid (to also be referred to as specific ketol fatty acid (III)), 9-hydroxy-12-oxo-10(E),15(Z)-octadecadienoic acid (to also be referred to as specific ketol fatty acid (IV)), 9,15,16-hydroxy-10-oxo-12(Z)-octadecamonoenoic acid, 9,15-hydroxy-10-oxo-16-chloro-octadecamonoenoic acid and 9,16-hydroxy-10-oxo-15-chloro-octadecamonoenoic acid.

The following indicates the chemical structural formulas of the specific ketol fatty acids (I) to (IV).

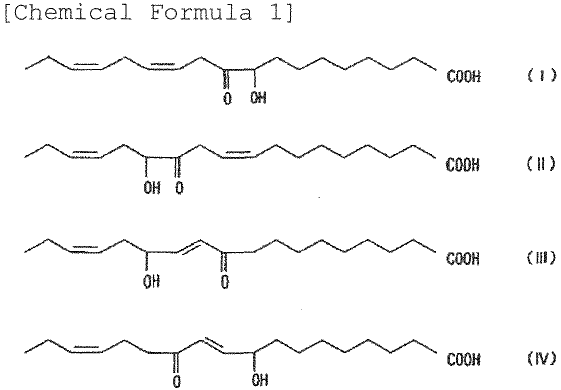

The chemical structural formulas of other specific ketol fatty acids along with the methods for synthesizing these specific ketol fatty acids are as disclosed in Patent Document 9.

Specific ketol fatty acids, and particularly KODA, have been indicated to demonstrate activation effects in the manner of growth regulatory effects, growth promotional effects and dormancy inhibitory effects on morning glories, digitalis, chrysanthemums, geraniums, fairy primrose, begonias, perpetual begonias, carnations, rice and strawberries (Patent Document 9), and are substances that exhibit a high degree of universality with respect to plant species.

In general, a limited number of plant hormones such as auxins, gibberellins, cytokinins, ethylene and abscisic acid are known to have various effects corresponding to their active sites. In the case of auxins, for example, although auxins promote growth of the stem and coleoptile, they inhibit growth of roots and lateral buds, while promoting the development of buds and fruit. On the other hand, although cytokinins inhibit stem and root elongation, they promote cell enlargement in true leaves and seed leaves. The specific ketol fatty acid of the present invention also demonstrates various effects corresponding to the active site thereof. Japanese Unexamined Patent Publication No. H11-29410 and Japanese Unexamined Patent Publication No. 2001-131006 describe that the specific ketol fatty acid of the present invention demonstrates various effects such as bud formation, promotion of plant growth, growth regulatory effects and dormancy inhibitory effects. The invention of the present application is based on having found that the specific ketol fatty acid also demonstrates effects that promote normal adventitious embryo differentiation from a callus in addition to the effects described above.

Since the specific ketol fatty acid of the present invention is known to demonstrate growth regulatory effects, growth promotional effects and dormancy inhibitory effects in numerous types of plant species, it also thought to have universality with respect to promotional effects on normal adventitious embryo differentiation from a callus. Thus, the effect of the specific ketol fatty acid of the present invention of promoting normal adventitious embryo differentiation from a callus is not limited to the Spathiphyllum, Peucedanum japonicum and tobacco plant described in the following examples.

Examples of plants in which the plant cell differentiation promoting agent of the present invention is used include angiosperms (dicotyledons and monocotyledons), pteridophytes and gymnosperms. Among angiosperms, examples of dicotyledons include plants of the Convolvulaceae family including plants belonging to the genus Datura (Datura stramonium), plants belonging to the genus Calystegia (Calystegia japonica, Calystegia hederacea, Calystegia soldanella), plants belonging to the genus Ipomoea (Ipomoea pes-caprae, Ipomoea batatas) and plants belonging to the genus Cuscuta (Cuscuta japonica, Cuscuta australis), plants of the Caryophyllaceae family such as plants belonging to the genus Dianthus, plants belonging to the genus Stellaria, plants belonging to the genus Minuartia, plants belonging to the genus Cerastium, plants belonging to the genus Caryophyllaceae, plants belonging to the genus Arenaria, plants belonging to the genus Moehringia, plants belonging to the genus Pseudostellaria, plants belonging to the genus Honckenya, plants belonging to the genus Spergula, plants belonging to the genus Trifolium, plants belonging to the genus Silene, plants belonging to the genus Lychnis, plants belonging to the genus Melandryum or plants belonging to the genus Cucubalus, as well as plants of the Casuarinaceae family, Saururaceae family, Piperaceae family, Chloranthaceae family, Salicaceae family, Myricaceae family, Juglandaceae family, Betulaceae family, Fagaceae family, Ulmaceae family, Moraceae family, Urticaceae family, Podostemaceae family, Proteaceae family, Olacaceae family, Santalaceae family, Loranthaceae family, Aristolochiaceae family, Mitrastemonaceae family, Balanophoraceae family, Polygonaceae family, Chenopodiaceous family, Amaranthaceae family, Nyctaginaceae family, Theligonaceae family, Phytolaccaceae family, Aizoaceae family, Portulacaceae family, Magnoliaceae family, Trochodendraceae family, Cercidiphyllaceae family, Nymphaeaceae family, Ceratophyllaceae family, Ranunculaceae family, Lardizabalaceae family, Berberidaceae family, Menispermaceae family, Calycanthaceae family, Lauraceae family, Papaveraceae family, Capparaceae family, Cruciferae family, Droseraceae family, Nepenthaceae family, Crassulaceae family, Saxifragaceae family, Goodeniaceae family, Hamamelidaceae family, Platanaceae family, Rosaceae family, Leguminosae family, Oxalidaceae family, Geraniaceae family, Linaceae family, Zygophyllaceae family, Rutaceae family, Simaroubaceae family, Meliaceae family, Polygalaceae family, Euphorbiaceae family, Callitrichaceae family, Buxaceae family, Empetraceae family, Coriariaceae family, Anacardiaceae family, Aquifoliaceae family, Celastraceae family, Staphylaceae family, Icacinaceae family, Aceraceae family, Hippocastanaceae family, Sapindaceae family, Sapiaceae family, Balsaminaceae family, Rhamnaceae family, Vitaceae family, Elaeocarpaceae family, Tiliaceae family, Malvaceae family, Sterculiaceae family, Actinidiaceae family, Theaceae family, Clusiaceae family, Elatinaceae family, Tamaricaceae family, Violaceae family, Flacourtiaceae family, Stachyuraceae family, Passifloraceae family, Begoniaceae family, Cactaceae family, Thymelaeaceae family, Elaeagnaceae family, Lythraceae family, Punicaceae family, Rhizophoraceae family, Alangiaceae family, Melastomataceae family, Trapaceae family, Onagraceae family, Haloragaceae family, Hippuridaceae family, Araliaceae family, Umbelliferae family, Cornaceae family, Diapensiaceae family, Clethraceae family, Pyrolaceae family, Ericaceae family, Myrsinaceae family, Primulaceae family, Plumbaginaceae family, Ebenaceae family, Symplocaceae family, Styracaceae family, Oleaceae family, Loganiaceae family, Gentianaceae family, Apocynaceae family, Asclepiadaceae family, Polemoniaceae family, Boraginaceae family, Verbenaceae family, Lamiaceae family, Solanaceae family (e.g., eggplants, tomatoes), Scrophulariaceae family, Bignoniaceae family, Pedaliaceae family, Orobanchaceae family, Gesneriaceae family, Lentibulariaceae family, Acanthaceae family, Myoporaceae family, Phrymaceae family, Plantaginaceae family, Rubiaceae family, Caprifoliaceae family, Adoxaceae family, Valerianaceae family, Dipsacaceae family, Cucurbitaceae family, Campanulaceae family and Asteraceae family.

Similarly, examples of monocotyledons include plants of the Azollaceae family including plants belonging to the genus Spirodela (Spirodela polyrhiza) and plants belonging to the genus Lemna (Lemna aoukikusa, Lemna trisulcata), plants of the Orchidaceae family including plants belonging to the genus Cattleya, plants belonging to the genus Cymbidium, plants belonging to the genus Dendrobium, plants belonging to the genus Phalaenopsis, plants belonging to the genus Ailuropoda, plants belonging to the genus Paphiopedilum and plants belonging to the genus Oncidium, as well as plants of the Typhaceae family, Sparganiaceae family, Potamogetonaceae family, Najadaceae family, Scheuchzeriaceae family, Alismataceae family, Hydrocharitaceae family, Triuridaceae family, Gramineae family (e.g., rice, barley, wheat, rye and corn), Cyperaceae family, Palmae family, Araceae family, Eriocaulaceae family, Commelinaceae family, Pontederiaceae family, Juncaceae family, Stemonaceae family, Liliaceae (e.g., asparagus), Amaryllidaceae family, Dioscoreaceae family, Iridaceae family, Musaceae family, Zingiberaceae family, Cannaceae family and Burmanniaceae family.

Although the aforementioned plants are considered to be plants in which the plant cell differentiation promoting agent of the present invention is used, the plant cell differentiation promoting agent of the present invention is preferably used particularly in plants in which seed-mediated propagation is difficult. Specific examples thereof include orchids, lilies, garlic, flowering trees, jatropha, aloe, agave, elephant tree, areca palm, dragon tree, xerographica, octopus tree, tulips, parlor palm, heart vine, ficus, philodendron, hedera, baby tears, peperomia, pothos, aralia, sea squirt, windowleaf, wire vine, purple heart, pachia, star jasmine, hypoestes, pilea, mosaic plant, poinsettia, prayer plant, hibiscus, bougainvillea, bridal veil, begonia, pachira, ficus, pumila, Benjamin tree, palms and yucca. In addition, the present technology is also useful during vegetative propagation of individuals having superior traits. There are no particular limitations on the plant species at that time.

The concentration at which the specific ketol fatty acid is used can be selected corresponding to the type of plant in which it is used. In the case of applying by dropping a solution containing the plant cell differentiation promoting agent directly onto a callus, the solution contains the specific ketol fatty acid at a concentration of 0.01 ppm to 10 ppm. Preferably, the concentration range of the specific ketol fatty acid can be an arbitrary range that combines an lower limit value of any of 0.01, 0.02, 0.03, 0.05, 0.1, 0.2 and 0.3 ppm and an upper limit value of any of 0.1, 0.2, 0.3, 0.5, 1.0, 1.5, 2.0, 3.0, 5.0, 7.0 and 10 ppm (provided that the lower limit value does not exceed the upper limit value). More preferably, the specific ketol fatty acid can be used at a concentration of 0.1 ppm to 3 ppm and even more preferably at a concentration of 0.03 ppm to 0.3 ppm. A person with ordinary skill in the art can determine the optimum concentration by carrying out a simple experiment corresponding to the plant species. In the case of adding to a medium, the specific ketol fatty acid is added to the medium so as to be within the aforementioned concentration ranges.

The callus used in the method for producing a redifferentiated plant body of the present invention is that which has been induced from a portion of a plant body, and may be obtained from a transformation system or by cell fusion. A conventionally known method can be used to induce a callus from a portion of a plant body. Namely, a callus can be obtained by culturing a portion of a plant body in a solid medium or liquid medium prepared by adding a nutrient source such as sucrose to a medium used in plant tissue culturing, such as Murashige and Skoog (MS) medium, Linsmaier and Skoog (LS) medium, Gamborg B5 medium, White's medium, Nitsch medium, Knudson C medium, SB medium, R2 medium, N6 medium or Tuleeke medium, followed by the addition of a plant hormone in the form of an auxin such as 2,4-dichlorophenoxyacetic acid or a cytokinin such as kinetin or benzyladenine. Culturing conditions for inducing a callus consist of static culturing or shake culturing at 15°C to 35°C in the presence or absence of light.

Although the induced callus is cultured by transplanting to subculture medium, it can also be transplanted to a differentiation medium to be subsequently described. Subculture medium is a medium for allowing the callus to grow while maintaining in an undifferentiated state, and may be any commonly used subculture medium. For example, the subculture medium is a medium obtained by adding sugars, inorganic salts, vitamins, auxins, and as necessary, cytokinins, amino acids and the like, to the aforementioned basal medium, and although a solid medium can be used, a liquid medium is preferable. The callus subculture medium may be the same as the callus induction medium. Culturing conditions are the same as culturing conditions for inducing a callus. Callus subculturing is preferably carried out every 1 to 4 weeks.

Differentiation medium refers to a medium that causes a callus maintained in an undifferentiated state to differentiate to a regenerated plant body by going through an adventitious embryo, adventitious root or adventitious bud. A conventionally known medium is used for the differentiation medium used in the present invention, and an example thereof is obtained by adding sugars, inorganic salts, vitamins, and as necessary, auxins, cytokinins or amino acids and the like, to the aforementioned basal medium. A solid medium or a liquid medium can be used for the differentiation medium. Examples of gelling agents used when preparing a solid medium include agar and gellan gum. Culturing conditions preferably consist of static culturing at 15°C to 35°C in the presence of light.

The plant cell differentiation promoting agent of the present invention can be used by adding to subculture medium and/or differentiation medium, or by dropping onto a callus on a solid medium. In the case of using the plant cell differentiation promoting agent in a solid medium, it may be preliminarily contained in a liquid medium prior to solidification, or may be added by coating onto the surface of a solid medium.

The formation of a normal adventitious embryo from a callus can be promoted by culturing a callus on a subculture medium or differentiation medium containing the plant cell differentiation promoting agent of the present invention or by dropping the plant cell differentiation promoting agent of the present invention onto the callus. In addition, the formation of an adventitious root or adventitious bud from a callus can also be promoted by culturing a callus on a subculture medium or differentiation medium containing the plant cell differentiation promoting agent of the present invention or by dropping the plant cell differentiation promoting agent of the present invention onto the callus. Whether an adventitious embryo, adventitious root or adventitious bud is formed depends on the ratio of the amounts of auxins and cytokinins present in the medium, and in general, adventitious root differentiation is observed if the amount of auxins is large, adventitious bud differentiation is observed if the amount of cytokinins is large, and an adventitious embryo is formed if the amount of auxins is decreased to an extremely low level. The differentiated adventitious embryo and adventitious bud are both able to differentiate into a regenerated plant body. In summary thereof, the plant cell differentiation promoting agent of the present invention can be said to be a callus differentiation promoting agent. Since differentiation is promoted by adding to a medium containing plant hormones such as auxins and cytokinins conventionally used to induce adventitious embryos from calluses, the plant cell differentiation promoting agent of the present invention can also be said to be a plant cell differentiation promotion assistant or callus differentiation promotion assistant.

On the other hand, the plant cell differentiation promoting agent of the present invention is also able to promote differentiation of an adventitious organ, and particularly an adventitious root or adventitious bud, from a plant section. Thus, the plant cell differentiation promoting agent of the present invention can be used in micropropagation methods, and can be used as a germination promoting agent or rooting promoter of a plant section. Although examples of the plant section in which the plant cell differentiation promoting agent of the present invention is used include any arbitrary part of a plant such as a leaf, stem or root, it can also be used to promote rooting of an auxiliary bud cut from a plant body.

The plant cell differentiation promoting agent of the present invention is able to demonstrate the differentiation promotional action thereof by adding to a subculture medium or differentiation medium during the time period from 3 weeks before transferring to the differentiation medium to 2 weeks after transferring to the differentiation medium. More preferably, the differentiation promotional action thereof can be demonstrated by adding to subculture medium or differentiation medium during the time period from 2 weeks before transplant to 1 week after transplant to the differentiation medium.

The plant cell differentiation promoting agent of the present invention is preferably used together with a plant hormone commonly used in plant tissue culturing. Although examples of plant hormones used together with the plant cell differentiation promoting agent of the present invention include auxins, cytokinins, gibberellins, ethylene and abscisic acid, auxins and cytokinins are preferable.

In another aspect of the present invention, the present invention relates to a medium containing a specific ketol fatty acid. The medium of the present invention is preferably a callus subculture medium or differentiation medium containing a specific ketol fatty acid. These media are the same as commonly used callus subculture media or differentiation media with the exception of containing a specific ketol fatty acid. Examples of basal media of commonly used callus subculture media or differentiation media include MS medium, LS medium, N6 medium, Gamborg B5 medium, White's medium, Nitsch medium, Knudson C medium, SB medium, R2 medium and Tuleeke medium, and these media can be used as differentiation media by further adding auxins and cytokinins to these basal media to obtain subculture media, followed by decreasing the amount or completely removing the auxins while changing the amount of cytokinins. These media may be liquid media or solid media.

Examples of auxins used in the present description include indoleacetic acid (IAA), indolebutyric acid (IBA), naphthalenic acid, naphthoxyacetic acid, phenylacetic acid, 2,4-dichlorophenoxyacetic acid (2,4-D) and 2,4,5-trichlorophenoxyacetic acid (2,4,5,-T). Examples of cytokinins used in the present description include zeatin, benzyladenine and thidiazuron.

A specific example of the specific ketol fatty acid contained in the plant cell differentiation promoting agent of the present invention in the form of 9-hydroxy-10-oxo-12(Z),15(Z)-octadecadienoic acid (KODA) can be produced using an extraction method by which it can be extracted from a species of plant of the Lemnaceae family in the form of Lemna aoukikusa. KODA can also be produced using other production methods such as an enzymatic method consisting of allowing an enzyme such as C9-specific lipoxygenase (LOX) or allene oxide synthase (AOS) to act an unsaturated fatty acid in the form of α-linolenic acid (generic name: cis-9,12,15-octadecatrienoic acid) in compliance with the fatty acid metabolic pathways within plant bodies, or a chemical synthesis method using ordinary, known chemical synthesis methods. These production methods are specifically described in Japanese Unexamined Patent Publication No. H11-29410 and Japanese Unexamined Patent Publication No. 2001-131006.

Although the following provides a more detailed explanation of the present invention through examples thereof, these examples do not limit the scope of the present invention.

### Examples

### Example 1: Induction of Callus from Spathiphyllum

### Cultured Seedling

Cultured seedlings subcultured under conditions of 25°C in a dark location (photosynthetic photon flux density: 5.7 µmole/m²/sec, day length: 16 hours) in a solid medium obtained by adding 3% sucrose and 0.8% agar (Wako Pure Chemical Industries, Tokyo, Japan) to MS (Murashige and Skoog) medium placed in a flat box (internal volume: 300 ml) manufactured by Asahi Techno Glass followed by adjusting the pH to 5.8 were used as cultured Spathiphyllum seedlings (variety: Double Take).

Sucrose (3%), 2,4-dichlorophenoxyacetic acid (4 ppm), benzyladenine (BA) (0.2 ppm), casein acid hydrolysate (100 ppm) and 5 mM 2-morpholinoethanesulfonic acid monohydrate (MES) were added to MS medium solidified with 0.8% agar to obtain a medium for callus induction and growth (to be referred to as "subculture medium", pH 5.8). Leaf sheaths were collected from the cultured seedlings during week 4 of subculturing by peeling from the stump and using a roughly 2 cm section containing the peeled portion (leaf sheath base) as an explant. The explants were then planted in a flat box containing the subculture medium (50 ml). When the seedlings were cultured for 8 weeks in a dark location at 25°C, calluses were induced at the peeled portions of the planted seedlings. Embryogenic calluses were selected by subculturing these calluses in 0.2 g/container each of subculture medium and culturing for 1 month. The embryogenic calluses were able to be maintained for 2 years while retaining adventitious embryo induction potency by subculturing to the subculture medium every 4 weeks.

0.05 g aliquots of the embryonic calluses were planted in 500 ml Erlenmeyer flasks containing 160 ml of MS liquid medium containing sucrose (1%), fructose (1.1%), BA (0.1 ppm) and MES (5 mM) (pH 5.8, to be referred to as "differentiation medium"), followed by shake culturing (80 rpm) for 6 weeks at 25°C in a dark location (day length: 16 hours, synthetic photon flux density: 22.8 µmole/m²/sec) to obtain adventitious embryos. 0.2 g aliquots of these adventitious embryos were implanted in MS medium containing sucrose (3%) and obtained by solidifying with agar (0.8%) (pH 5.8, to be referred to as "germination medium") followed by culturing for 8 weeks at 25°C in a dark location (day length: 16 hours, photosynthetic photon flux density: 5.7 µmole/m²/sec) to cause the adventitious embryos to germinate and obtain complete plants.

### Example 2: Effect of KODA on Differentiation from Spathiphyllum Callus to Adventitious Embryo

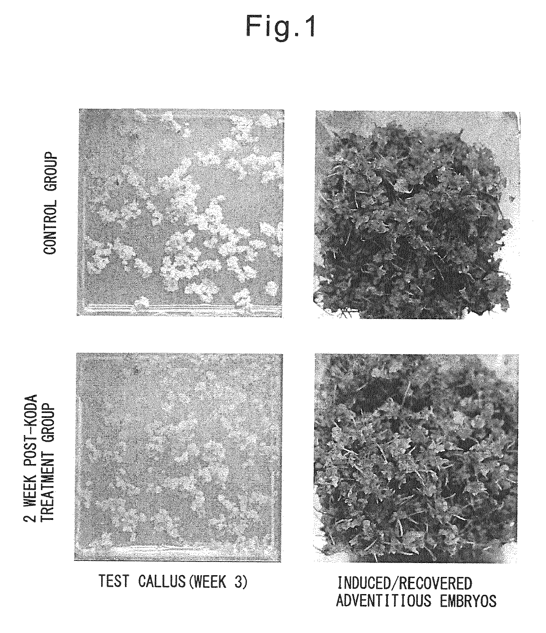

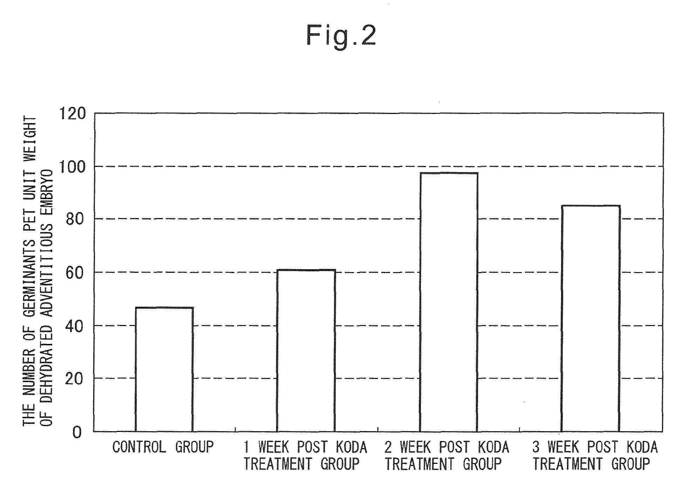

Embryogenic calluses subcultured in subculture medium were subcultured in fresh subculture medium followed by dropping a 3 ppm aqueous solution of KODA onto the calluses after 1 week (7 days), 2 weeks (14 days) or 3 weeks (20 days). The cultured calluses were planted in liquid differentiation medium 3 weeks after the start of subculturing, and the differentiated adventitious embryos were recovered 6 weeks later (FIG. 1), dehydrated and weighed after dehydrating. The adventitious embryos were then planted in germination medium and the numbers of resulting germinants were counted. The results are shown in Table 1. The number of germinants per 1 g of dehydrated adventitious embryos was calculated from the measurement results (FIG. 2). Although there were no changes observed in the 1 week post-treatment group in comparison with the control group, the number of germinants increased the greatest in the 2 week post-treatment group, while that in the 3 week post-treatment group decreased to a lower number than the 2 week post-treatment group. The effect of KODA on inducing adventitious embryos in calluses was therefore thought to be specific to the time at which it is applied.
[Table 1]

**Table 1 Effect of KODA Treatment on Induction of Germinants from Calluses**

| | Dehydrated adventitious embryo weight per flask (g) | No. of germinants per flask | No. of germinants per unit dehydrated adventitious embryo weight |
|---|---|---|---|
| Control group | 9.5 | 444 | 47 |
| 1 week post-treatment group | 7.1 | 431 | 61 |
| 2 week post-treatment group | 7.2 | 702 | 98 |
| 3 week post-treatment group | 6.8 | 578 | 85 |

### Example 3: Effect of KODA Concentration on Differentiation from Spathiphyllum Callus to Adventitious Embryo

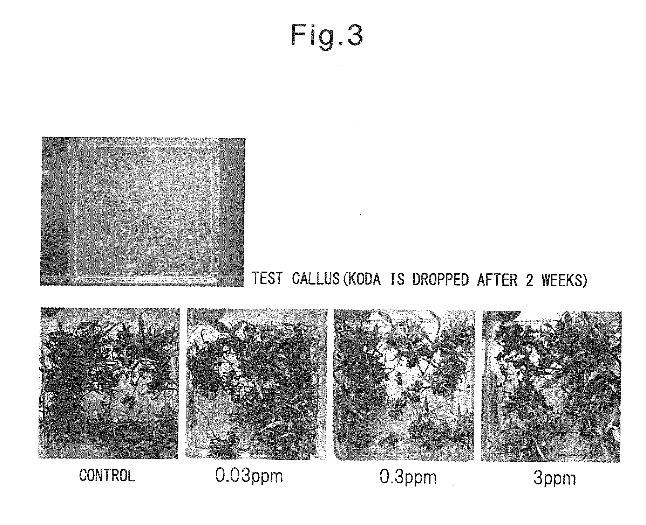

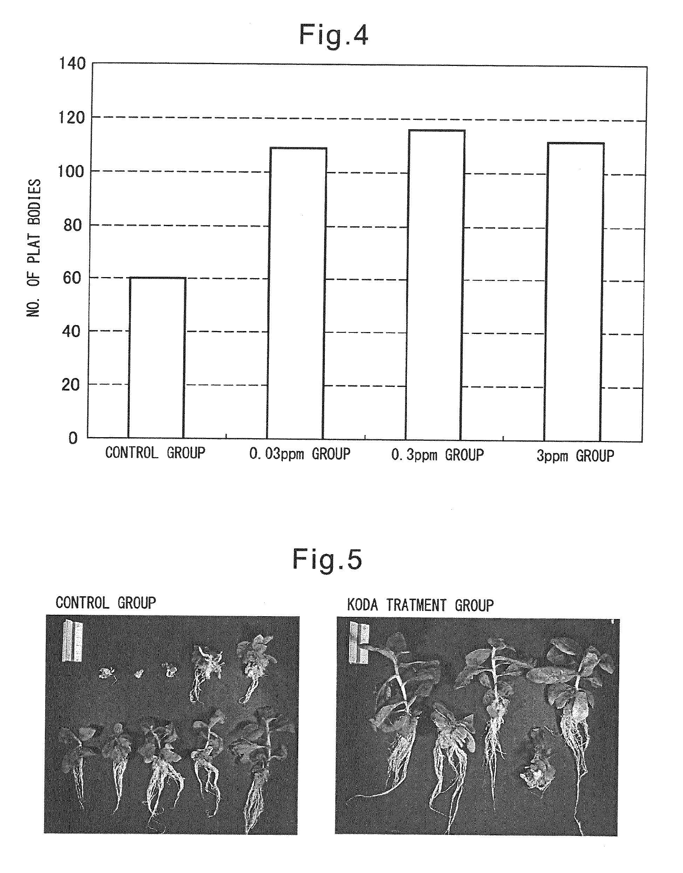

Embryogenic calluses subcultured in subculture medium were planted in fresh differentiation medium followed by dropping a 0.03 ppm, 0.3 ppm or 3 ppm aqueous solution of KODA onto the calluses and recording the number of normally regenerated plant bodies 8 weeks later. Although plant bodies were regenerated in all of the test groups, a large number of plant bodies were observed to exhibit symptoms of considerable stress (narrow leaves) (FIG. 3). These plant bodies exhibiting symptoms of considerable stress were not included in the number of normally regenerated plant bodies. There were numerous plant bodies exhibiting symptoms of stress in the control group, and as a result thereof, the number of normally regenerated plant bodies was low. The results are shown in FIG. 4.

### Example 4: Effect of KODA on Differentiation from Callus in Peucedanum japonicum

Peucedanum japonicum seeds from Yonaguni Island in Okinawa Prefecture were planted in artificial soil (Metro-Mix 360, Sun Grow Horticulture Canada Ltd.) and allowed to germinate under conditions of 20°C and a 12-hour light-dark cycle. Two months later, the seedling plants were taken out, only the above ground portion was washed well with water, and after disinfecting with 70% alcohol (10 seconds) and a 10-fold dilution of sodium hypochlorite (7 minutes), the seedling plants were rinsed four times with pure water. The petiols were cut out and a cut measuring about 5 mm in length was aseptically made therein followed by planting in MS medium (Wako Pure Chemical Industries, solidified with 0.7% agar) containing 2,4-dichlorophenoxyacetic acid (0.022 mg/L) and kinetin (10.75 mg/L) solidified with 0.7% agar. The petiols were cultured for 2 months under conditions of 25°C and a light-dark cycle of 12 hours to grow greening tissue masses containing incomplete leaf tissue. Two months later, the greening tissue masses were immersed in 1 µM KODA (KODA treatment group) or water (control group) , and placed on MS medium (solidified with 0.7% agar) to which benzyladenine (BA) was added to a concentration of 2 ppm with indoleacetic acid (IAA) to a concentration of 0.1 ppm, 0.5 ppm or 1 ppm. The results are shown in Table 2. As can be understood from the table, although a large number of differentiated greening plant bodies were obtained from greening tissue masses immersed in KODA, only a small number of differentiated plant bodies were obtained in the control group (water) in the case of 1.0 ppm indoleacetic acid + 2.0 ppm benzyladenine.

**[Table 2]**

| Hormone conditions | IAA (0.1 ppm) + BA (2.0 ppm) | | IAA (0.5 ppm) + BA (2.0 ppm) | | IAA (1.0 ppm) + BA (2.0 ppm) | |
|---|---|---|---|---|---|---|
| KODA treatment | Control group | KODA treatment group | Control group | KODA treatment group | Control group | KODA treatment group |
| No. of redifferentiated greening plant bodies | 0 | 29 | 0 | 20 | 8 | 27 |

### Example 5: Effect of KODA on Differentiation from Tobacco Callus

After washing tobacco (Nicotiana tabacum) seeds with 70% ethanol, the surfaces of the seeds were sterilized for 3 minutes with 5% sodium hypochlorite and 0.05% Tween 20 followed by washing 5 times with sterilized water. After washing, the seeds were immersed in an aqueous KODA solution (100 µM) followed by planting the washed seeds on solid MS medium (Wako Pure Chemical Industries) containing 3% sucrose, 0.8% agar (Wako Pure Chemical Industries) and 2,4-dichlorophenoxyacetic acid (Kanto Chemical, 3 ppm) with kinetin (Nakarai Tesque, 0.1 ppm) and adjusted to pH 5.7 to induce calluses for 3 weeks under conditions of 25°C and a light-dark cycle of 16 hours.

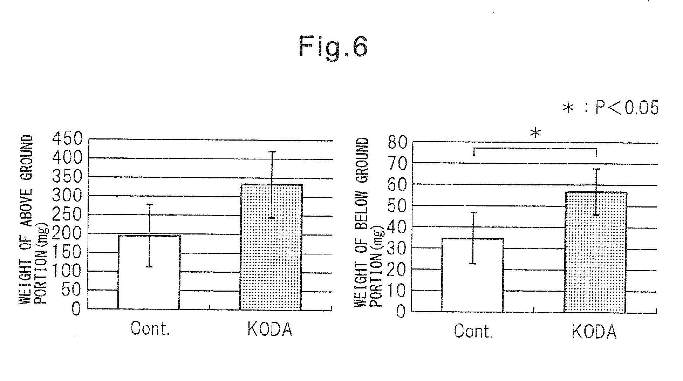

The above ground portions of the tobacco seedlings that underwent callus induction were removed, and only the root portions in which calluses were present were treated by immersing in 100 µM KODA (KODA treatment group) or water (control group) followed by inducing differentiation of the above ground portions under conditions of 25°C and a light-dark cycle of 16 hours in solid differentiation medium obtained by adding 3% sucrose, 0.8% agar (Wako Pure Chemical Industries), 1-naphthalenic acid (Nacalai Tesque, 0.2 ppm) and 6-benzyladenine (Nacalai Tesque, 2 ppm) to MS medium contained in plastic Petri dishes and adjusting to pH 5.7. Culturing in the differentiation medium was carried out by transferring to fresh differentiation medium after culturing for 2 weeks, namely for a total of 4 weeks. The resulting above ground portions were excised with a scalpel. The excised above ground portions were immersed in 100 µM aqueous KODA solution (KODA treatment group) or water (control group) followed by planting it in MS medium (100 mL) contained in culture bottles (CB-3, As One), which is prepared by adding sucrose (3%) to MS medium, and adjusting pH to 5.7, and is solidified with 0.8% agar, and then cultured for 4 weeks under conditions of 25°C and light-dark cycle of 16 hours so as to differentiate below ground portions. Following completion of culturing, the plant bodies were removed from the culture bottles, excess medium was removed, and images of the entire cultured plants were photographed, the results of which are shown in FIG. 5. In addition, after photographing, only normal individuals were selected and divided into above ground portions and below ground portions. The divided biological samples were subjected to freeze-drying and then weighed after removing moisture (FIG. 6).

Since individuals having abnormalities such as reduced size frequently result during individual regeneration in the case of tissue culturing, a comparison was made of the number of normal individuals among the redifferentiated individuals between a control group and a KODA treatment group. As a result, in contrast to normal individuals only accounting for 30% of the redifferentiated individuals in the control group, in the KODA treatment group, 60% of the redifferentiated individuals were normal individuals. A comparison was then made of the growth of the redifferentiated individuals based on their dry weights (FIG. 6). Although there was no significant difference at a level of significance of 5% between the two groups with respect to the above ground portions, weight was clearly observed to demonstrate an increasing trend based on a comparison of average values. In addition, with respect to the below ground portions, a significant difference was confirmed at a level of significance of 5%, and growth of the below ground portions was clearly demonstrated to have been significantly promoted. On the basis of the above, it was clearly demonstrated that, in tissue cultured individuals, KODA tends to increase the normalization rate in individual differentiation of plants mediated by a callus, and that it primarily acts to significantly promote root growth.

## Claims

1. A plant cell differentiation promoting agent, comprising a ketol fatty acid having 4 to 24 carbon atoms, wherein a carbon atom that composes a carbonyl group and a carbon atom that is bound to a hydroxyl group are in the α position.

2. The plant cell differentiation promoting agent according to claim 1, wherein carbon-carbon double bonds are present at 1 to 6 locations in the ketol fatty acid (provided that the number of double bonds does not exceed the number of carbon bonds of the ketol fatty acid).

3. The plant cell differentiation promoting agent according to claim 1 or 2, wherein the number of carbon atoms of the ketol fatty acid is 18, and carbon-carbon double bonds are present at 2 locations.

4. The plant cell differentiation promoting agent according to any one of claims 1 to 3, wherein the ketol fatty acid is 9-hydroxy-10-oxo-12(Z),15(Z)-octadecadienoic acid.

5. The plant cell differentiation promoting agent according to any one of claims 1 to 4, wherein the plant cell differentiation promoting agent promotes differentiation from a plant callus to an adventitious embryo, an adventitious root or an adventitious bud.

6. The plant cell differentiation promoting agent according to any one of claims 1 to 4, wherein the plant cell differentiation promoting agent promotes differentiation from a plant section to an adventitious bud or an adventitious root.

7. A method for producing a regenerated plant from a callus, comprising a step for applying the plant cell differentiation promoting agent according to any one of claims 1 to 4 to a callus during the time period from 3 weeks before transplanting the callus maintained in a subculture medium to 2 weeks after transplanting the callus to a differentiation medium.

8. A plant culture medium containing the plant cell differentiation promoting agent according to any one of claims 1 to 4.
